# EUROPÄISCHE PATENTSCHRIFT

(11) **EP 0 857 733 B1**
(45) Veröffentlichungstag und Bekanntmachung des Hinweises auf die Patenterteilung: **09.07.2003**
(21) Anmeldenummer: 97121124.8
(22) Anmeldetag: 02.12.1997
(51) Int. Cl.: A61K 38/42

(54) **Mit Schutzliganden der Sauerstoffbindungsstellen versehene Hämoglobine als künstliche Sauerstoffträger zur direkten biologisch-medizinischen Anwendung und Verfahren zu ihrer Herstellung**
Hemoglobin having protecting ligands of oxygen binding positions as artificial oxygen carriers for direct biological or medicinal use and process for its preparation
Hémoglobine ayant des ligands protecteurs des positions de liaison de l'oxygène comme porteur d'oxygène artificiel pour utilisation biologique-médicale directe et procédé pour sa préparation

(30) Priorität: 15.01.1997 DE 19701037
(43) Veröffentlichungstag der Anmeldung: 12.08.1998
(73) Patentinhaber: Sangui BioTech AG, 58455 Witten (DE); Pötzschke, Harald, Dr., 65207 Wiesbaden-Breckenheim (DE)
(72) Erfinder: Barnikol, Wolfgang, Prof.Dr.Dr., 55128 Mainz (DE); Pötzschke, Harald, 65207 Wiesbaden (DE)
(74) Vertreter: Müller, Claudia

(56) Entgegenhaltungen:
- WO-A-94/22482

## Beschreibung

Die Erfindung betrifft die Verwendung künstlicher Sauerstoffträger aus nativen oder modifizierten Hämoglobinen, die bis zu ihrer Anwendung andauernd zuverlässig vor Funktionsverlust geschützt sind, die vor ihrer Anwendung keiner weiteren Behandlun bedürfen und deren Reaktivierung nach Beginn der Anwendung steuerbar ist, zur Versorgung eines biologisch- medizinischen Systems.

Künstliche Sauerstoffträger auf Basis von Hämoglobinen werden für viele Zwecke benötigt, sie können aber im Verlaufe ihrer Herstellung und Lagerung teilweise oder vollständig ihre Funktionsfähigkeit verlieren. Dies zu verhindern ist notwendig, damit künstliche Sauerstoffträger aus Hämoglobinen möglichst lange lagerfähig und verwendbar bleiben.

Allgemein werden zur Herstellung künstlicher Sauerstoffträger verschiedene Wege beschritten, einer davon ist die Herstellung geeignerter Lösungen nativer oder chemisch modifizierter Hämoglobine (zum Stand der Forschung und Entwicklungen siehe "Issues from Vth International Symposium on Blood Substitutes, San Diego/CA, USA, March 1993", *Artificial Cells, Blood Substitutes, and Immobilization Biotechnology* **22** (1994), Heft 2 - Heft 4). Ein Problem der Handhabbarkeit solcher pharmazeutischer Zubereitungen als künstliche Sauerstoffträger ist ihre zunehmende Inaktivierung durch spontane Oxidation zum Methämoglobin, welches keinen Sauerstoff mehr transportieren kann. Dies betrifft sowohl die eigentliche Herstellung beim Produzenten, als auch die folgende Lagerung bis zur Anwendung.

Es gibt eine Reihe von Verfahren und Methoden dieses Problem zu beseitigen. Entweder verringern sie das Ausmaß der Oxidation des Hämoglobins, oder das oxidierte Hämoglobin wird wieder reduziert.

Eine Möglichkeit einer weitgehenden Vermeidung spontaner Oxidation besteht darin, das Hämoglobin zu desoxygenieren (d. h. den Sauerstoff in der Zubereitung völlig zu entfernen), denn Desoxyhämoglobin wird deutlich langsamer zu Methämoglobin oxidiert als Oxyhämoglobin (ANTONINI et al. (1981) (Hrsg.) : *Methods in Enzymology, Volume 76, Hemoglobin*, Academic Press, New York u. a. 1981). Die Schwierigkeit dieser Vorgehensweise liegt in der Notwendigkeit den Sauerstoff wirklich vollständig zu entfernen, denn verbleibende Reste wirken weiter, und zwar wird teilweise oxygeniertes Hämoglobin noch wesentlich schneller oxidiert als völlig oxygeniertes (BROOKS J. (1935) : "The Oxidation of Haemoglobin to Methaemoglobin by Oxygen. II. The Relation between the Rate of Oxidation and the Partial Pressure of Oxygen", *Proceedings of the Royal Society (London), Series B* **118** : 560 - 570).

Weiter ist es wegen der Temperaturabhängigkeit der Oxidationsgeschwindigkeit möglich, das Ausmaß der Oxidation durch Lagerung und/oder Präparation bei niedrigst möglicher Temperatur (für wäßrige Lösungen also bei etwa 4 °C) zu minimieren. Dies erfordert aber die (aufwendige) permanente Bereithaltung technischer Geräte (Kühlschränke) und hat den weiteren Nachteil, daß die Zubereitung vor einer Anwendung erwärmt werden muß.

Außerdem ist die Oxidationsgeschwindigkeit des Hämoglobins von der Wasserstoffionen-Konzentration, also dem pH-Wert, abhängig. Ein Minimum besteht z. B. für natives menschliches Hämoglobin im Intervall zwischen den pH-Werten 7,5 und 9,5 (SUGAWARA Y. et al. (1993) : "Autoxidation of Human Hemoglobin: Kinetic Analysis of the pH-Profile", *Japanese Journal of Physiology* **43** : 21 - 34). Messungen an einigen Polymeren menschlichen Hämoglobins (sogenannte Hyperpolymere) haben gezeigt, daß dies prinzipiell auch für diese gilt. Die Rückstellung des pH-Wertes vor einer Anwendung ist aber problematisch.

Auch der Zusatz gewisser Alkohole kann die Oxidation des Hämoglobins vermindern (NEDJARARROUME N. et al. (1993). : "Stabilizing Effect of Water Alcohol Solvents Towards Autoxidation of Human Haemoglobin", *Biotechnology and Applied Biochemistry* **18** : 25 - 36). Einige von ihnen wirken bereits in geringer Konzentration. Ein Problem hierbei ist die Toxizität dieser Alkohole.

Gewisse Metallionen (Cu2+, Fe3+ etc.) katalysieren die spontane Oxidation des Hämoglobins. Sie können durch Komplexierung mit z. B. EDTA (Ethylendiamin-Tetraessigsäure) unwirksam gemacht werden, allerdings fördert EDTA selbst die spontane Oxidation von Hämoglobin.

Spezifische Möglichkeiten des Schutzes künstlicher Sauerstoffträger vor Oxidation bestehen in der Zugabe reduzierender Substanzen. Diese bewirken unter Umständen sogar eine Re-Aktivierung oxidierten Hämoglobins.

Erstens ist eine chemische Reduktion möglich. Eine Re-aktivierung erfordert aber die Umsetzung mit einem starken Reduktionsmittel (z. B. mit Dithionit : BAUER und PACYNA, (1975) : "The Conversion of Trivalent to Divalent Iron in Hemoglobin of Various Species", *Analytical Biochemistry* **65** : 445 - 448). Wird ein Reduktionsmittel nach der Umsetzung entfernt, stellt sich das Problem der Oxidation des Hämoglobins erneut. Wird ein Reduktionsmittel bleibend zugesetzt, wird es selbst mit appliziert, was die Auswahl an geeigneten Stoffen stark vermindert. Geeignet könnte beispielsweise Ascorbinsäure sein (TOMODA (1978) : "Mechanism of Methemoglobin Reduction by Ascorbic Acid under Anaerobic Conditions", *Journal of Biological Chemistry* **253** : 7420 - 7423). Sie ist aber nur ein relativ schwaches Reduktionsmittel und somit nur zur Verhinderung einer weiteren Oxidation geeignet.

Hämoglobin kann auch enzymatisch reduziert werden. Für analytische Zwecke sind einige Möglichkeiten für geeignete Enzyme und Substrate beschrieben (HAYASHI et al. (1973) : "An Enzymic Reduction System for Metmyoglobin and Methemoglobin, and Ist Application to Functional Studies of Oxygen Carrier", *Biochimica et Biophysica Acta* **310** : 309 - 316, und ROSSI-FANELLI und ANTONINI (1958) : "Studies on the Oxygen and Carbon Monoxide Equilibria of Human Myoglobin", *Archives of Biochemistry and Biophysics* **77** : 478 - 492). Problematisch ist die Mit-Applikation dieser Proteine, so daß auf diesem Wege nur ein Oxidationsschutz im Verlauf der Herstellung der künstlichen Sauerstoffträger praktikabel ist.

Eine bislang für sämtliche Schritte der Präparation von Hämoglobinen bekannte Methode zur Minimierung des Ausmaßes ihrer Oxidation ist ihre Ligandierung mit Kohlenmonoxid, also ihre Überführung in Carbonylhämoglobin (oder Carboxyhämoglobin). Zum Schutz der zur Anwendung vorgesehenen Zubereitungen künstlicher Sauerstoffträger ist dieses Verfahren bislang jedoch nie vorgesehen gewesen, denn eine Reihe von Argumenten scheinen eine Anwendung zum angegebenen Zweck zu verbieten: Kohlenmonoxid wird als Ligand erheblich fester an die Sauerstoffbindungsstelle des Hämoglobins gebunden als Sauerstoff. Deshalb steht Carbonylhämoglobin praktisch nicht für einen Sauerstofftransport zur Verfügung. Carbonylhämoglobin ist deshalb für den Arzt "vergiftetes" Hämoglobin, da der Anteil des Hämoglobins, der als Carbonylhämoglobin vorliegt, für einen Sauerstofftransport ausfällt. Bei schweren Vergiftungen mit Kohlenmonoxid ist dazu oft eine akute Lebensgefährdung des Patienten vorhanden und die Blockierung der Sauerstofftransportfunktion muß therapiert werden. In experimentellen Arbeiten zum Nachweis der Wirksamkeit und Wirkungen künstlicher Sauerstoffträger aus Hämoglobinen werden die entsprechenden, mit Kohlenmonoxid ligandierten Hämoglobine sogar als akut nicht-funktionsfähige "Träger" eingesetzt (z. B. CONOVER et al. (1996) "Evaluation of the Oxygen Delivery Ability of PEG-Hemoglobin in Sprague-Dawley Rats during Hemodilution" (Abstract), *Artificial Cells, Blood Substitutes, and Immobolization Biotechnology* **24** : 325). Eine bleibende Ligandierung künstlicher Sauerstoffträger aus Hämoglobinen mit Kohlenmonoxid zum Schutz vor Oxidation scheint deshalb als nicht praktikabel zu sein.

Der Erfindung liegt die Aufgabe zugrunde, die vorhandene Funktionsfähigkeit künstlicher Sauerstoffträger aus Hämoglobinen während ihrer Herstellung und anschließenden Lagerung bis zu ihrer Anwendung weitestmöglich zu erhalten, ohne daß vor ihrer Anwendung weitere Behandlungschritte erforderlich sind.

Diese Aufgabe wird dadurch gelöst, daß man die Sauerstoffbindungsstelle mit einem geeigneten Schutzliganden versieht. Überraschend wurde gefunden, daß eine Überführung des erfindungsgemäßen künstlichen Sauerstoffträgers in eine Schutzliganden-freie Form vor seiner biologisch-medizinischen Anwendung nicht erforderlich ist.

Die gestellte Aufgabe wird erfindungsgemäß vorzugsweise dadurch gelöst, daß das Hämoglobin vollständig in Carbonylhämoglobin überführt und in dieser Form sowohl gelagert wird als auch so als künstlicher Sauerstoffträger ohne weitere Behandlung zur Anwendung kommt. Der Schutz erfolgt durch eine gegebenenfalls früh im Verlauf der Synthese durchzuführende Ligandierung mit Kohlenmonoxid (es entsteht Carboxy- (oder Carbonyl-) hämoglobin), die, als Wesensmerkmal der Erfindung, vor einer Applikation nicht rückgängig gemacht wird. Vielmehr erfolgt die Anwendung des künstlichen Sauerstoffträgers in dieser Form.

Die technisch äußerst aufwendige und kurzfristig nicht durchführbare Entfernung des Liganden vor einer Applikation kann erfindungsgemäß aus folgenden Gründen unterbleiben:
1.Im Blutgefäßsystem eines Organismus oder exzidierter Organe oder in technischen Vorrichtungen zur Kultur von Zellen zerfällt der applizierte Kohlenmonoxid-Hämoglobin-Komplex, gemäß der Hämoglobin-Kohlenmonoxid-Bindungskurve, wegen des im Blutplasma oder in der Nährlösung sehr niedrigen Partialdrucks des Kohlenmonoxids. Das frei werdende Kohlenmonoxid wird abgeatmet oder mit dem abgeführten Versorgungsgas entfernt. Das zunächst unmittelbar zu einem Sauerstofftransport nicht befähigte Hämoglobin wird so - durch De-Ligandierung - aktiviert.
2. Bei einer Anwendung in einem Organismus ist das in der pharmazeutischen Zubereitung des Carboxy-Hämoglobins nicht an Hämoglobin gebundene, frei gelöste Kohlenmonoxid, welches zugleich appliziert wird, zwar prinzipiell fähig, mit dem eigenen Hämoglobin des Empfängers Carboxyhämoglobin zu bilden. Seine Menge kann aber durch geeignete Wahl des Partialdruckes des Kohlenmonoxids in der Zubereitung vernachlässigbar gering gehalten werden. Die Hämoglobin-Kohlenmonoxid-Bindungskurve erlaubt einen vollständigen Umsatz des künstlichen Sauerstoffträgers aus Hämoglobinen zur karbonylierten Form (zum Carboxyhämoglobin) bereits bei sehr niedrigen Partialdrücken. Kohlenmonoxid ist ein natürlich vorkommendes Gas.

Weiterhin wurde gefunden, daß ein geschützter künstlicher Sauerstoffträger nach Verabreichung durch eine allmähliche Aktivierung gekennzeichnet ist, wobei die Geschwindigkeit der Reaktivierung - innerhalb gewisser Grenzen - durch Änderung der inspiratorischen Volumenfraktion oder des Partialdruckes des Sauerstoffes steuerbar ist.

Carboxyhämoglobin ist bekanntermaßen äußerst stabil gegen spontane Oxidation, auch während einer Lagerung bei Raumtemperatur, so daß als erzielbarer Vorteil die erfindungsgemäßen Präparate über lange Zeit ohne nachteilige Folgen für ihre Funktion als künstliche Sauerstoffträger aufbewahrt werden können.

Darüber hinaus kann der Prozeß des Zerfalls der Hämoglobin-Kohlenmonoxid-Komplexe des künstlichen Sauerstoffträgers im Blut durch eine Erhöhung des Anteils des Sauerstoffes im Inspirationsgas (Erhöhung der inspiratorischen Sauerstoff-Volumenfraktion), wie es zur Therapie z. B. auch der Vergiftung mit Kohlenmonoxid angewendet wird, gefördert und die Geschwindigkeit des Prozesses durch das Ausmaß der Erhöhung der inspiratorischen Sauerstoff-Volumenfraktion gesteuert werden.

Ein weiterer wesentlicher Vorteil einer Anwendung der erfindungsgemäß vor Funktionsverlust geschützten Hämoglobine als künstliche Sauerstoffträger ergibt sich aus der Möglichkeit - angepaßt an die Erfordernisse des einzelnen Patienten oder der speziellen Anwendung - ihre Funktion allmählich frei zu geben. So kommt es nach einer therapeutischen Gabe in einen Organismus nicht zu einer vorübergehenden relativen Überversorgung minder-durchbluteter Organe mit Sauerstoff; die funktionell an eine Hypoxie adaptierten Gewebe werden so vor einem oxidativen Schock mit toxischen Sauerstoffschäden (sogenannte Reperfusionsschäden) bewahrt.

Eine vorteilhafte Herstellung eines künstlichen Sauerstoffträgers aus Hämoglobinen sieht vor, diesen möglichst frühzeitig im Verlauf seiner Herstellung mit Kohlenmonoxid in mindestens ausreichender Konzentration, z. B. zunächst mit reinem Kohlenmonoxid, zu äquilibrieren, und so möglichst vollständig in Carboxyhämoglobin zu überführen. Auf diese Weise ist das verwendete Hämoglobin auch gegen partielle Oxidation sicher geschützt. Dieser Zustand wird im weiteren Verlauf der Präparationen aufrecht erhalten. Als abschließender Schritt wird dann - um den Gehalt frei gelösten Kohlenmonoxids zu minimieren und zugleich die vollständige Ligandierung des Hämoglobins mit Kohlenmonoxid zu erhalten - mit Kohlenmonoxid geringstmöglicher Konzentration (z. B. mit einem Volumenteil Kohlenmonoxid und 99 Volumenteilen Stickstoff) äquilibriert und die Zubereitung in diesem Zustand gasdicht (z. B. in Glasampullen oder Infusionsflaschen) zur Lagerung und nachfolgenden Applikation verpackt.

Als Schutzliganden der Sauerstoffbindungsstelle des Hämoglobins kommen auch andere dafür in der Literatur beschriebene Stoffgruppen (ANTONINI E. et al (Hrsg.) (1981): *Methods in Enzymology Volume 76* - *Hemoglobins* (COLOWICK S.P., KAPLAN N.O. (Hrsg.) *Methods in Enzymology*) Academic Press, New York u.a. 1981) in Betracht. Ein wie Kohlenmonoxid in Reinform gasförmiger geeigneter Schutzligand der Sauerstoffbindungsstelle ist Stickstoffmonoxid (NO). Als nicht-gasförmige kompetitive Liganden sind "Derivate" des Stickstoffmonoxids, wie Nitrosoverbindungen, und Isocyanide, jeweils in der Form bestimmter geeigneter Verbindungen, einsetzbar. Ihre sich dem Fachmann bekannte, leicht sich erschließende oder durch einfache Versuche feststellbare Eignung als Schutzligand für solche künstliche Sauerstoffträger aus Hämoglobinen hängt von ihrer Affinität zur Sauerstoffbindungsstelle sowie ihrer Verträglichkeit für den menschlichen Organismus oder die zu behandelnde Zellkultur oder Präraration ab.

Die Auswahl bestimmter Schutzliganden kann auf die mit ihrem Einsatz gewünschten Ergebnisse abgestellt werden. Beispielsweise ist es möglich, durch eine gleichzeitige Benutzung verschiedener Schutzliganden mit unterschiedlicher Affinität zu Hämoglobin eine steuerbare (abgestufte) Aktivierung des künstlichen Sauerstoffträgers während seiner Anwendung zu erreichen.

## Patentansprüche

1. Verwendung eines künstlichen Sauerstoffträger aus nativen oder modifizierten Hämoglobinen, wobei die Sauerstoffbindungsstellen mit Schutzliganden versehen sind, zur Herstellung eines Mittels für eine direkte Anwendung in einem biologisch - medizinischen System, **dadurch gekennzeichnet, dass** die Sauerstoffbindungsstellen der Sauerstoffträger mit einem reversibel bindenden Schutzliganden versehen sind und diese Sauerstoffträger zur direkten Versorgung des biologisch-medizinischen Systems mit Sauerstoff eingesetzt werden.

2. Verwendung gemäß Anspruch 1, **dadurch gekennzeichnet, dass** der Schutzligand Kohlenmonoxid ist.

3. Verwendung gemäß einem der Ansprüche 1 oder 2 **dadurch gekennzeichnet, dass** als Hämoglobin natürliches oder chemisch modifiziertes Hämoglobin menschlichen oder tierischen Ursprungs eingesetzt wird.

## Claims

1. Use of an artificial oxygen carrier consisting of native or modified haemoglobins, in which the oxygen binding sites are provided with protecting ligands, for the preparation of an agent for direct use in a biological-medicinal system, **characterized in that** the oxygen binding sites of the oxygen carriers are provided with a reversibly binding protecting ligand and that these oxygen carriers are used for supplying the biological-medicinal system directly with oxygen.

2. Use according to Claim 1, **characterized in that** the protecting ligand is carbon monoxide.

3. Use according to Claim 1 or 2, **characterized in that** the haemoglobin used is natural or chemically modified haemoglobin of human or animal origin.

## Revendications

1. Utilisation d'un transporteur d'oxygène artificiel constitué d'hémoglobine naturelle ou modifiée dans laquelle les sites de liaison de l'oxygène sont munis de ligands protecteurs pour la préparation d'un produit destiné à être utilisé directement dans un système biologique - médical, **caractérisée en ce que** les sites de liaison de l'oxygène du transporteur d'oxygène sont munis de ligands protecteurs à liaison réversible et ce transporteur d'oxygène est utilisé pour l'alimentation directe du système biologique - médical en oxygène.

2. Utilisation selon la revendication 1, **caractérisée en ce que** le ligand protecteur est le monoxyde de carbone.

3. Utilisation selon l'une des revendications 1 ou 2, **caractérisée en ce que** l'on utilise comme hémoglobine une hémoglobine naturelle ou chimiquement modifiée d'origine humaine ou animale.
